# EUROPEAN PATENT APPLICATION

(11) **EP 2 199 291 A1**
(43) Date of publication of application: **23.06.2010**
(21) Application number: 08171964.3
(22) Date of filing: 17.12.2008
(51) Int. Cl.: C07D 403/04, A61K 31/501, A61P 9/00

(54) **Process for preparation of pimobendan hydrochloride**

(71) Applicant: Novartis AG, 4056 Basel (CH)
(72) Inventor: Reven, Sebastjan, 4270 Jesenice (SI); Ham, Zoran, 1420 Trbovlje (SI)
(74) Representative: Liphardt, Bernd

(57) **Abstract**

The present invention refers to the process for preparation of pimobendan hydrochloride, to a crystalline form of pimobendan hydrochloride, and to pharmaceutical compositions comprising it.

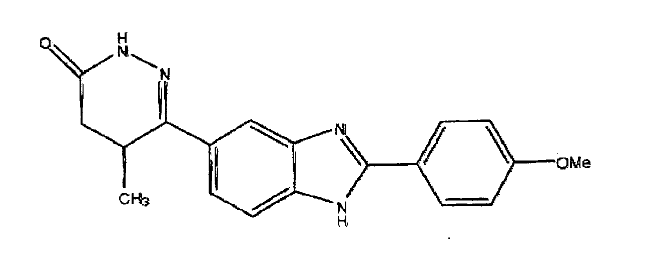

## Description

The present invention refers to the process for preparation of pimobendan hydrochloride, to a crystalline form of pimobendan hydrochloride, and to pharmaceutical compositions comprising it.

Pimobendan, (4,5-dihydro-6-(2-(4-methoxyphenyl)-1H-benzimidazol-5-yl)-5-methyl-3(2H))-pyridazinone, is a benzimidazole derivative with cardiotonic, hypotensive and anti-thrombotic effect. It is commercially available as chewing tablets or capsules under the brand name Vetmedin® (Boehringer Ingelheim) and is in particular useful in veterinary medicine. Pimobendan is a cardiac pharmaceutical, termed an "inodilator" because it possesses both positive inotropic and balanced peripheral vasodilation properties. Unlike historical positive inotropes (e.g. digoxin, milrinone) that function by increasing intracellular calcium concentrations, resulting in increased cardiac energy and oxygen requirements, pimobendan acts as a positive inotrope principally by enhancing the affinity of myocardial troponin C to existing intracellular calcium. Peripherally, pimobendan is a phosphodiesterase III (PDE III) inhibitor, resulting in balanced peripheral vasodilation through increased efflux of intracellular calcium from vascular smooth muscle. Additional properties include reversal of desensitization of baroreceptors, improved cardiac relaxation (lusitropy), reduced platelet aggregation, and an anti-inflammatory effect mediated through favourable cytokine modulation.

Pimobendan, its acid addition salts, its preparation and its use in therapeutics have been disclosed in Patent specification DE 2922336. Methods for preparation of pimobendan hydrochloride (Compound of formula (I)) described in DE 2922336 involve the use of organic solvents (isopropyl alcohol, methanol) and ethereal hydrochloric acid.

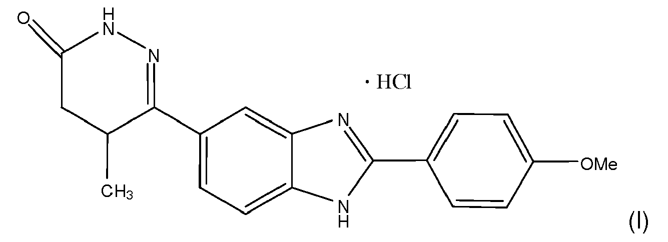

The main drawback of such methods is associated with the residuals of organic solvents and the necessity of preparation of solution of hydrochloric acid in diethyl ether by bubbling gaseous HCl (taken from a gas cylinder or generated from NaCl) through cold diethyl ether under anhydrous conditions. This is a tedious and skill-demanding procedure due to the necessity of ensuring strictly anhydrous conditions.

Therefore, the objective of the invention is to provide a process for preparation of pimobendan hydrochloride that overcomes the abovementioned drawback and results in a well defined, perfectly reproducible crystalline form of pimobendan hydrochloride that especially exhibits valuable characteristics as filtration, drying and ease of formulation.

In one aspect present invention relates to a process for preparation of pimobendan hydrochloride.

In another aspect present invention relates to a crystalline form of pimobendan hydrochloride In another aspect present invention relates to pharmaceutical compositions comprising such obtained pimobendan hydrochloride.

In another aspect present invention relates to use of such obtained pimobendan hydrochloride for the treatment of cardiovascular diseases, viruses and viral diseases.

In another aspect the present invention relates to a process for the preparation of pimobendan hydrochloride comprising the steps of
(i) suspending pimobendan in the aqueous solution of hydrochloride acid
(ii) stirring and heating the suspension
(iii) cooling the suspension
(iv) separating from the suspension said pimobendan hydrochloride

The suspension of pimobendan is carried out in aqueous solution of hydrochloride acid wherein concentration of hydrochloride acid is preferably between 0.01M and 1.0M, more preferably between 0.01M and 0.1M.

Suspension of pimobendan in the aqueous solution of hydrochloride acid is heated at a temperature between 30 to 50 ºC for at least 30 minutes under stirring, preferably at a temperature between 35 to 45 ºC for at least 30 minutes under stirring.

Subsequently the suspension is cooled to a temperature between 0 ºC to 30 ºC, preferably between 20 ºC to 25 ºC.

Pimobendan hydrochloride may be separated by techniques well known in the art, e.g. filtration, centrifugation, decanting.

The precipitate is filtered, washed with water and optionally dried in an oven at elevated temperature and under vacuum (40 °C).

In another aspect present invention relates to pimobendan hydrochloride in crystalline form. In another aspect the present invention relates to pimobendan hydrochloride in crystalline form which is characterized by the X-ray diffraction pattern with characteristic diffraction peaks at the angles 2θ of ± 0.2: 6,6751; 9,4759; 10,7452; 13,2318; 17,0785; 18,6736; 21,1005; 24,3423; 25,6239; 27,1334.

In another preferred aspect the present invention relates to pimobendan hydrochloride in crystalline form which is characterized by the X-ray diffraction pattern with characteristic diffraction peaks at the angles 2θ of ± 0.2.

| No. | Pos. [°2Th.] | d-spacing [A] | Rel. Int. [%] |
|---|---|---|---|
| 1 | 6,6751 | 13,24211 | 8,03 |
| 2 | 9,4759 | 9,33357 | 63,83 |
| 3 | 10,7452 | 8,23366 | 23,08 |
| 4 | 13,2318 | 6,69143 | 14,87 |
| 5 | 17,0785 | 5,19195 | 11,87 |
| 6 | 18,6736 | 4,75189 | 19,67 |
| 7 | 21,1005 | 4,21053 | 33,52 |
| 8 | 24,3423 | 3,65663 | 73,87 |
| 9 | 25,6239 | 3,47658 | 100,00 |
| 10 | 27,1334 | 3,28650 | 59,64 |

In another preferred aspect the present invention relates to pimobendan hydrochloride in crystalline form which is characterized by the X-ray diffraction pattern with characteristic diffraction peaks at the angles 2θ of ± 0.2.

| No. | Pos. [°2Th.] | d-spacing [A] | Rel. Int. [%] |
|---|---|---|---|
| 1 | 6,6751 | 13,24211 | 8,03 |
| 2 | 9,4759 | 9,33357 | 63,83 |
| 3 | 10,7452 | 8,23366 | 23,08 |
| 4 | 13,2318 | 6,69143 | 14,87 |
| 5 | 17,0785 | 5,19195 | 11,87 |
| 6 | 18,6736 | 4,75189 | 19,67 |
| 7 | 21,1005 | 4,21053 | 33,52 |
| 8 | 22,3441 | 3,97891 | 20,13 |
| 9 | 24,3423 | 3,65663 | 73,87 |
| 10 | 25,6239 | 3,47658 | 100,00 |
| 11 | 27,1334 | 3,28650 | 59,64 |
| 12 | 29,7180 | 3,00629 | 18,67 |

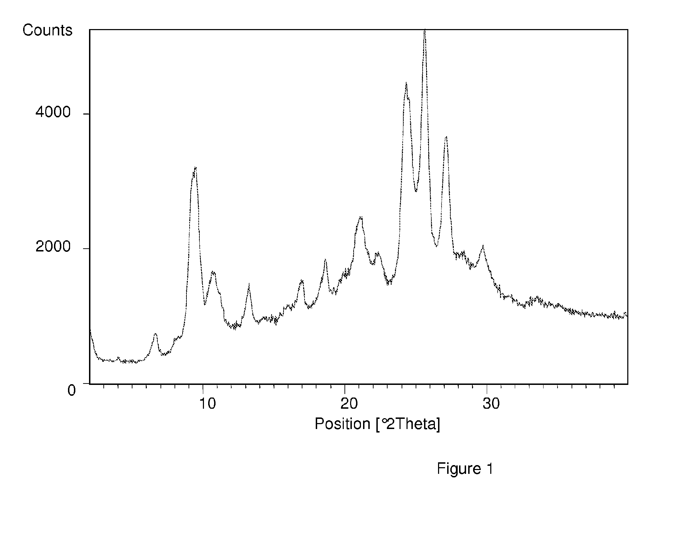

In another preferred aspect the present invention relates to pimobendan hydrochloride in crystalline form having a powder X-ray powder diffraction pattern substantially such as in Figure 1.

In another aspect the present invention relates to pimobendan hydrochloride in crystalline form which is characterized by the IR spectrum with peaks at 1667, 1607, 1499, 1469, 1374, 1345, 1305, 1268, 1189, 1128, 1080, 1022, 952, 924, 880, 837, 792, 770, 737, 710, 695, 669 cm⁻¹_{.}

In another preferred aspect the present invention relates to pimobendan hydrochloride in crystalline form which is characterized by the IR spectrum with peaks at 3709, 3308, 2642, 1667, 1607, 1499, 1469, 1374, 1345, 1305, 1268, 1189, 1128, 1080, 1022, 952, 924, 880, 837, 792, 770, 737, 710, 695, 669 cm⁻¹.

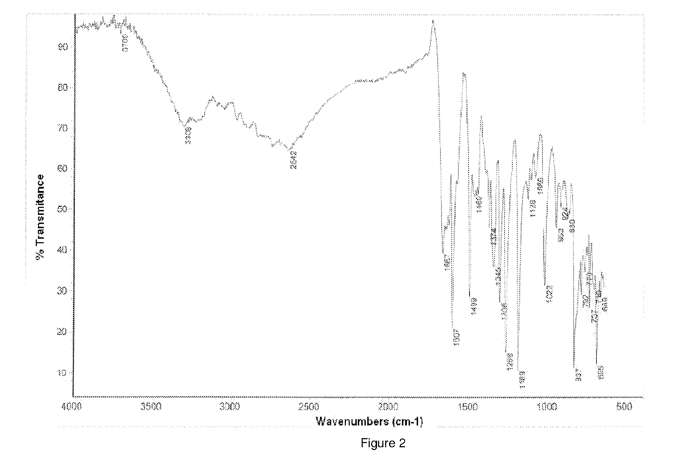

In another preferred aspect the present invention relates to pimobendan hydrochloride in crystalline form having IR spectrum substantially such as in Figure 2.

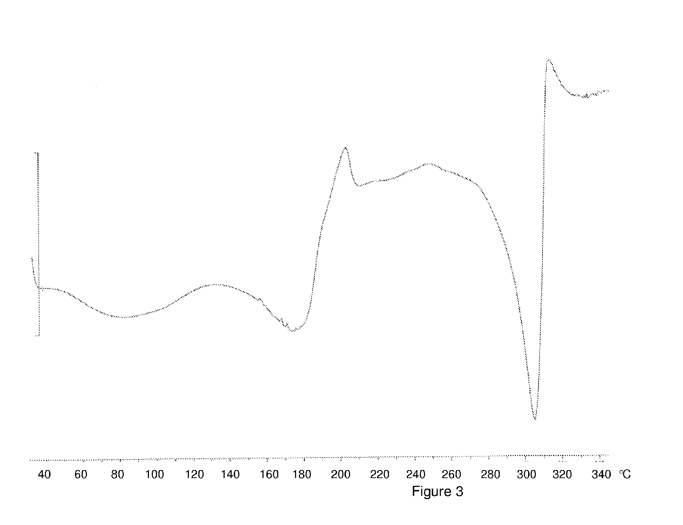

In another aspect the present invention relates to pimobendan hydrochloride in crystalline form which is characterized by the DSC thermogram with the melting peak at around 305 °C. In preferred another aspect the present invention relates to pimobendan hydrochloride in crystalline form having DSC thermogram substantially such as in Figure 3.

In another aspect the present invention relates to pimobendan hydrochloride in crystalline form which is characterized by the Raman spectrum with peaks at 1626, 1607, 1500, 1572, 1264 cm⁻¹_{.}

In another preferred aspect the present invention relates to pimobendan hydrochloride in crystalline form which is characterized by the Raman spectrum with peaks at. 1626, 1607, 1572, 1500, 1452, 1377, 1348, 1264, 1195, 1130, 1084, 955, 793, 137 cm⁻¹.

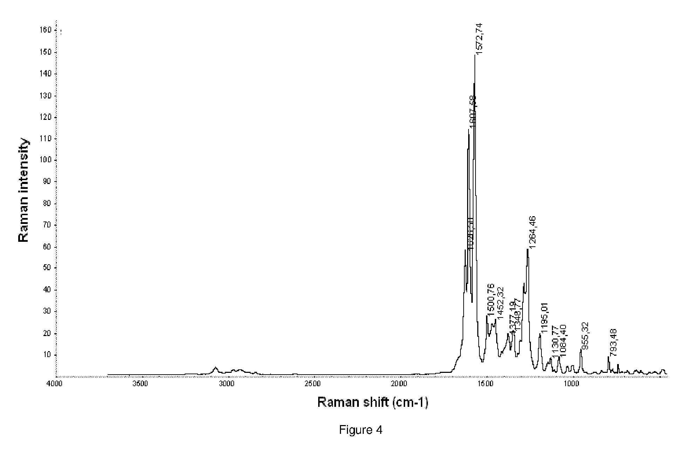

In another aspect the present invention relates to pimobendan hydrochloride in crystalline form having Raman spectrum substantially such as in Figure 4.

In another aspect present invention relates to pharmaceutical composition comprising pimobendan hydrochloride in combination with one or more pharmaceutically acceptable excipients wherein said pimobendan hydrochloride is prepared by the above mentioned process.

The present invention also relates to pharmaceutical compositions comprising pimobendan hydrochloride in crystalline form in combination with one or more pharmaceutically acceptable excipients.

The pharmaceutical composition of the present invention can be any form of pharmaceutical composition known in the field of the pharmaceutical technology. Preferably, the pharmaceutical composition is in the form of tablets, pills, capsules, caplets, lozenges, sachets or powder. Tablets may be suitably coated (film coated tablets, pills). A pharmaceutical composition according to the present invention is more preferably in form of tablets.

The pharmaceutical composition of the present invention can be prepared by the processes which are conventional in pharmaceutical technology.

In another aspect present invention relates to use of pimobendan hydrochloride for the treatment of cardiovascular diseases, viruses and viral diseases wherein said pimobendan hydrochloride is prepared by above mentioned process.
- Figure 1:: X-ray powder diffraction pattern of pimobendan hydrochloride in crystalline form
- Figure 2:: IR spectrum of pimobendan hydrochloride in crystalline form
- Figure 3:: DSC thermogram of pimobendan hydrochloride in crystalline form
- Figure 4:: Raman spectrum of pimobendan hydrochloride in crystalline form

Following representative example further explains the invention

### Example 1:

100 mg of pimobendan was suspended in 100 ml of aqueous solution of hydrochloride acid (0.1 M) at ambient temperature. Suspension was heated to 40 °C under stirring for 30 minutes. Subsequently the suspension was cooled to a room temperature (ca. 20 - 25 °C). The precipitate was filtered, washed with water and dried in an oven at 40 ºC and under vacuum. 90 mg of pure pimobendan hydrochloride in a crystalline form was obtained.

### Example 2:

100 mg of pimobendan was suspended in 100 ml of aqueous solution of hydrochloride acid (0.01 M) at ambient temperature. Suspension was heated to 40 °C under stirring for 30 minutes. Subsequently the suspension was cooled to a room temperature (ca. 20 - 25 °C). The precipitate was filtered, washed with water and dried in an oven at 40 ºC and under vacuum. 69 mg of pure pimobendan hydrochloride in a crystalline form was obtained.

The product obtained according to the Example 1 was analyzed by following methods:

### X-Ray powder diffraction method:

Conditions for obtaining powder X-ray diffraction (XRD) pattern: The powder x-ray diffraction patterns were obtained by methods known in the art using Philips X'Pert PRO diffractometer with X'Celerator detector using CuKα radiation (tube operating at 45 kV and 40 mA) in the Bragg-Brentano (reflection) geometry. Data were recorded from 2 to 40 °2θ in steps of 0.033 °2θ and the measurement time of 50 seconds per step. Variable divergence and antiscatter slits were used to maintain 12 mm of sample length irradiated.

| No. | Pos. [°2Th.] | d-spacing [A] | Rel. Int. [%] |
|---|---|---|---|
| 1 | 6,6751 | 13,24211 | 8,03 |
| 2 | 9,4759 | 9,33357 | 63,83 |
| 3 | 10,7452 | 8,23366 | 23,08 |
| 4 | 13,2318 | 6,69143 | 14,87 |
| 5 | 17,0785 | 5,19195 | 11,87 |
| 6 | 18,6736 | 4,75189 | 19,67 |
| 7 | 21,1005 | 4,21053 | 33,52 |
| 8 | 22,3441 | 3,97891 | 20,13 |
| 9 | 24,3423 | 3,65663 | 73,87 |
| 10 | 25,6239 | 3,47658 | 100,00 |
| 11 | 27,1334 | 3,28650 | 59,64 |
| 12 | 29,7180 | 3,00629 | 18,67 |

### IR spectroscopy method:

Conditions for obtaining infrared spectrum: Fourier transform infrared (FTIR) spectra were recorded with a Nicolet Nexus spectrometer. Spectra over a range of 4000 to 400 cm⁻¹ with a resolution of 2 cm⁻¹ (16 scans) were recorded on KBr tablets.
3709, 3308, 2642, 1667, 1607, 1499, 1469, 1374, 1345, 1305, 1268, 1189, 1128, 1080, 1022, 952, 924, 880, 837, 792, 770, 737, 710, 695, 669 cm⁻¹

### Differential Scanning Calorimetry:

Conditions for obtaining DSC thermogram: Thermograms were obtained with Mettler Toledo DSC822^{e} differential scanning calorimeter. The sample (4-6 mg) was placed in an unsealed aluminium pan with a hole and heated at 10 °C/min in the temperature range from 30°C to 350°C. Melting point temperature around 305 °C

### Raman Spectroscopy:

Raman spectra were obtained with Thermo Nicolet Nexus, FT Raman module spectrometer. Spectra over a range of 4000 to 500 cm -1 were recorded: 1626, 1607, 1572, 1500, 1452, 1377, 1348, 1264, 1195, 1130, 1084, 955, 793, 137 cm⁻¹.

## Claims

1. Process for the preparation of pimobendan hydrochloride comprising the steps of
(i) suspending pimobendan in the aqueous solution of hydrochloride acid
(ii) stirring and heating the suspension
(iii) cooling the suspension
(iv) separating said pimobendan hydrochloride.

2. Process according to previous claim further comprising drying of pimobendan hydrochloride.

3. Process according to claim 1 wherein concentration of hydrochloride acid is between 0.01M and 0.1M

4. Process according to claim 1 wherein heating is performed at a temperature comprised between 30 to 50 ºC for at least 30 minutes

5. Process according to claim 1 wherein the suspension is cooled to a temperature between 0 ºC and 30 ºC.

6. Pimobendan hydrochloride obtained by the process according to any one of claims 1-5.

7. Pimobendan hydrochloride according to claim 6 **characterized by** the X-ray diffraction pattern with characteristic diffraction peaks at the angles 2θ of ± 0.2: 6,6751; 9,4759; 10,7452; 13,2318; 17,0785; 18,6736; 21,1005; 24,3423; 25,6239; 27,1334.

8. Pimobendan hydrochloride according to claim 7 **characterized by** the IR spectrum with peaks at 1667, 1607, 1499, 1469, 1374, 1345, 1305, 1268, 1189, 1128, 1080, 1022, 952, 924, 880, 837, 792, 770, 737, 710, 695, 669 cm⁻¹.

9. Pimobendan hydrochloride according to any one of claims 7 - 8 **characterized by** the Raman spectrum with peaks at 1626, 1607, 1572, 1500, 1452, 1377, 1348, 1264, 1195, 1130, 1084, 955, 793, 137 cm⁻¹.

10. Pimobendan hydrochloride according to any one of claims 7 - 9 **characterized by** the DSC thermogram with a melting peak at around 305 °C.
